# EUROPEAN PATENT APPLICATION

(11) **EP 4 537 751 A1**
(43) Date of publication of application: **16.04.2025**
(21) Application number: 23820006.7
(22) Date of filing: 12.05.2023
(51) Int. Cl.: A61B 5/145, A61B 5/00, G16H 10/60

(54) **BLOOD SUGAR MEASUREMENT DEVICE AND METHOD**

(30) Priority: 10.06.2022 KR 20220070876
(71) Applicant: i-Sens, Inc., Seoul 06646 (KR)
(72) Inventor: LEE, Jaesook, Seoul 06646 (KR); LEE, Seungchun, Seoul 06646 (KR); PARK, Hyoseon, Seoul 06646 (KR); OH, Sukyong, Seoul 06646 (KR)
(74) Representative: Weickmann & Weickmann PartmbB
(86) International application number: PCT/KR2023/006481
(87) International publication number: WO 2023/239070

(57) **Abstract**

A blood glucose measurement device having a display, includes a housing having one or more openings on one side, one or more contact electrodes configured to electrically coupled to a sensor strip inserted into the housing through the one or more openings, one or more control buttons configured to turn on the blood glucose measurement device, and one or more processors configured to control a first execution screen to be displayed in response to receiving a first signal by the one or more contact electrodes and, display a second execution screen in response to receiving a second signal by the one or more control buttons.

## Description

### Technical Field

The disclosure relates to a blood glucose measurement device and method.

### Background Art

Diabetes has a major impact on the daily lives of patients. Diabetics should continually monitor their diet to maintain blood glucose balance and check whether insulin intake is appropriate for their diet. Currently, various portable devices for measuring blood glucose levels have been studied and developed. Typically, a blood glucose measurement device may measure a blood glucose value by collecting blood through a separately prepared needle and placing the blood on a sensor strip of the blood glucose measurement device. Recently, a blood glucose value is continuously measured by invasively inserting and fixedly attaching a needle-type sensor into a part of a human body. However, various embodiments described in the disclosure are not limited to such a blood glucose measurement device.

Recently, various application programs have been developed for patients to directly monitor changes in blood glucose values. For example, a patient may install a program to monitor a blood glucose value on a user terminal and record and check blood glucose values collected hourly. In addition, to perform monitoring tasks using the user terminal, communication between a blood glucose measurement device and the user terminal must be established, and thus, some blood glucose measurement devices are equipped with antennas for wireless communication.

### Disclosure

### Technical Problem

The disclosure provides a communication method between a blood glucose measurement device and an external terminal, or a control method based on the communication method.

### Technical Solution

According to an embodiment, a blood glucose measurement device having a display includes a housing having one or more openings on one side, one or more contact electrodes configured to electrically couple with a sensor strip inserted into the housing through the one or more openings, one or more control buttons configured to turn on the blood glucose measurement device, and a processor configured to control a first execution screen to be displayedin response to receiving a first signal by the one or more contact electrodes and, display a second execution screen in response to receiving a second signal by the one or more control buttons.

In an embodiment, the blood glucose measurement device may further include a control pad configured to receive a control input, and the processor may be further configured to display, on the display, a third execution screen in response to receiving one or more control inputs through the control pad while the second execution screen is displayed, wherein the third execution screen may be identical to the first execution screen or include a user interface representation to output the first execution screen.

In an embodiment, the first execution screen may include a first user interface representation to collect one or more pieces of information for diabetes diagnosis, and the second execution screen may include a second user interface representation including a menu to switch to a plurality of user interface representations.

In an embodiment, the blood glucose measurement device may further include a control pad configured to receive a control input, wherein the processor may be further configured to generate blood glucose information based on at least one of one or more additionally-input control inputs and one or more sensing values detected by using the sensor strip after the first execution screen or the second execution screen is displayed, and display, on the display, a fourth execution screen indicating the blood glucose information.

In an embodiment, the blood glucose measurement device may further include one or more antennas, wherein the processor may be further configured to activate the one or more antennas in response to the display of the fourth execution screen on the display.

In an embodiment, the blood glucose measurement device may further include one or more antennas, wherein the processor may be further configured to control the one or more antennas to transmit a message requesting feedback to an external device in response to opening of an electrical coupling between the sensor strip and the one or more contact electrodes.

In an embodiment, the processor may be further configured to store blood glucose information obtained by using the sensor strip in a memory, include, to the message, the blood glucose information stored in the memory and transmit the message to the external device, and exclude, from the memory, the blood glucose information transmitted to the external device.

In an embodiment, the processor may be further configured to store blood glucose information obtained by using the sensor strip in a memory, select at least a portion of the blood glucose information stored in the memory based on at least one of an obtaining time of the blood glucose information or a transmission status of the blood glucose information, and include, to the message, the selected portion of the blood glucose information and transmit the message to the external device.

According to an embodiment, a blood glucose measurement device including a display includes a housing having one or more openings on one side, one or more induction electrodes configured to electrically couple with an introducer inserted into the housing through the one or more openings, one or more control buttons configured to turn on the blood glucose measurement device, one or more glucose sensors configured to continuously or periodically obtaining blood glucose information, and one or more processors configured to control a first execution screen to be displayed in response to receiving a first signal generated between the one or more induction electrodes and the introducer, and display a second execution screen in response to receiving a second signal by the one or more control buttons.

In an embodiment, the display may include a touch sensor configured to receive a control input, and the one or more processors may be further configured to display, on the display, a third execution screen in response to receiving one or more control inputs through the touch sensor while the second execution screen is displayed, wherein the third execution screen may be identical to the first execution screen or include a user interface representation to output the first execution screen.

In an embodiment, the first execution screen may include a first user interface representation to collect one or more pieces of information for diabetes diagnosis, and the second execution screen may include a second user interface representation including a menu to switch to a plurality of user interface representations.

In an embodiment, the display may include a touch sensor configured to receive a control input, and the one or more processors may be further configured to obtain blood glucose information based on at least one of one or more additionally-input control inputs and one or more sensing values detected by using the one or more glucose sensors after the first execution screen or the second execution screen is displayed, and display, on the display, a fourth execution screen indicating the blood glucose information.

In an embodiment, the blood glucose measurement device may further include one or more antennas, and the one or more processors may be further configured to activate the one or more antennas in response to the display of the fourth execution screen on the display.

In an embodiment, the blood glucose measurement device may further include one or more antennas, and the one or more processors may be further configured to store, in a memory, one or more pieces of blood glucose information obtained by the one or more glucose sensors, and include, to a message requesting feedback, at least some of the pieces of blood glucose information stored in the memory and transmit the message to an external device.

In an embodiment, the one or more processors may be further configured to exclude, from the memory, the blood glucose information transmitted to the external device.

In an embodiment, the one or more processors may be further configured to select at least some of the one or more pieces of blood glucose information stored in the memory, based on an obtaining time of the blood glucose information or a transmission status of the blood glucose information, and include, to a message requesting feedback, only the selected blood glucose information and transmit the message to the external device.

In an embodiment, the one or more openings may be formed to penetrate the housing in a moving direction of the introducer.

According to an embodiment, a blood glucose measurement method performed by a blood glucose measurement device including a housing having one or more openings on one side, one or more contact electrodes configured to electrically couple with a sensor strip inserted into the housing through the one or more openings, and one or more control buttons configured to turn on the blood glucose measurement device, includes displaying a first execution screen in response to receiving a first signal by the one or more contact electrodes, and displaying a second execution screen in response to receiving a second signal by the one or more control buttons.

### Description of Drawings

FIG. 1 is a block diagram of a blood glucose measurement system according to an embodiment.
FIG. 2 is a block diagram of a blood glucose measurement device according to an embodiment.
FIGS. 3A and 3B show implementation examples of a blood glucose measurement device.
FIG. 4 is a flowchart illustrating a blood glucose measurement method according to an embodiment.
FIG. 5 is a flowchart illustrating a blood glucose measurement method according to another embodiment.
FIGS. 6A and 6B are diagrams illustrating an example of a process of activating one or more components of a blood glucose measurement device based on an interaction with an external element.
FIG. 7 is a flowchart for describing operation S430 of FIG. 4 and operation S530 of FIG. 5.
FIGS. 8A and 8B are diagrams illustrating an example of a process of transmitting a feedback message request to an external device based on an interaction with an external element.
FIG. 9 is a sequence diagram of a blood glucose measurement method according to an embodiment.
FIG. 10 is a diagram illustrating an example of transmission of a feedback message request including sensing information.
FIG. 11 is a diagram illustrating an example of a case where transmission of a feedback message request was successful.
FIG. 12 is a diagram illustrating an example of a case where transmission of a feedback message request has failed at least once.
FIG. 13 is a diagram illustrating an example of transmission of a feedback message request including user information.
FIG. 14 is a diagram illustrating an example of a process of switching a user interface representation initiated by a first interaction by a control pad and/or a touch screen panel.
FIG. 15 is a diagram illustrating a comparative example of a process of switching a user interface representation initiated by a second interaction by an insertion of an external element.
FIG. 16 is a diagram illustrating an example of a process of switching a user interface representation initiated by removal of an external element.

### Mode for Invention

Hereinafter, the disclosure will be described in detail by explaining embodiments of the disclosure with reference to the accompanying drawings so that those skilled in the art may easily implement the disclosure. However, the disclosure may be implemented in various forms, and embodiments described herein are not limited thereto. In the description of the disclosure, like reference numerals in the drawings denote like elements. In addition, in the drawings and related descriptions, explanations of well-known functions and configurations may be omitted for clarity and brevity.

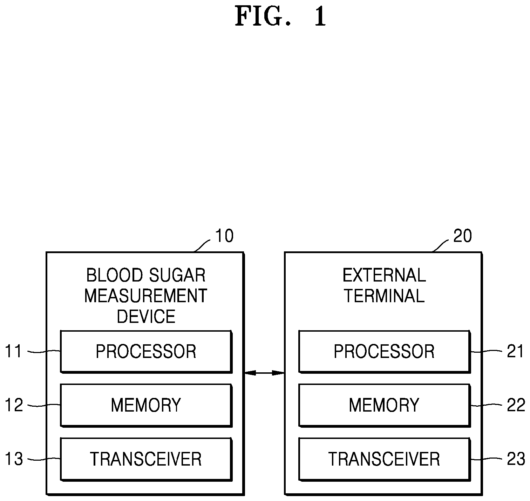

FIG. 1 is a block diagram of a blood glucose measurement system according to an embodiment.

According to an embodiment, a blood glucose measurement system may include a blood glucose measurement device 10 and an external terminal 20. The blood glucose measurement device 10 and the external terminal 20 may be communicatively connected via a wired or wireless network.

The blood glucose measurement device 10 refers to a device that detects blood glucose components within a body and may output the blood glucose components as blood glucose values. The blood glucose measurement device 10 may measure blood glucose concentration through an electrochemical method, a reflectance photometry method, a micro-needle method, or the like according to a measurement principle, and the blood glucose measurement device 10 applied to the disclosure is not limited any of the above.

The external terminal 20 refers to a terminal capable of receiving a user input or providing a user with information. The external terminal 20 includes, for example, a smartphone, a laptop computer, a wearable device, and a server, but is not limited to thereto. The external terminal 20 may be communicatively or electrically connected to the blood glucose measurement device 10 and may output various pieces of information through a connected output unit (e.g., a display, a speaker, a vibrator, or the like) (not shown). For example, the external terminal 20 connected to the blood glucose measurement device 10 may output information related to blood glucose values received from the blood glucose measurement device 10 through a display. The information related to blood glucose values is not limited to blood glucose values and may further include diagnostic information determined based on at least one of the user's health status, habits, meal times, meal contents, and underlying diseases. The term "external terminal" may be interchangeably used with "external device."

According to an embodiment, the blood glucose measurement device 10 and the external terminal 20 may be implemented as electronic devices capable of communicating via a network. Here, the electronic devices may include one or more processors 11 and 21, one or more memories 12 and 22, and one or more transceivers 13 and 23. The processors 11 and 21, the memories 12 and 22, and the transceivers 13 and 23 may be electrically connected to each other and may exchange signals with each other. The memories 12 and 22 store instructions for executing a control method according to an embodiment, and the processors 11 and 21 may implement operations according to various embodiments by using the instructions stored in the memories 12 and 22.

According to an embodiment, the processors 11 and 21 may include a storage and processing circuit unit for supporting operations of an electronic device according to an embodiment. The storage and processing circuit unit may include storage such as non-volatile memory (e.g., flash memory or other electrically programmable read-only memory (ROM) configured to form a solid-state drive (SSD)) and volatile memory (e.g., static or dynamic random-access memory (RAM)). Processing circuit units within the processors 11 and 21 may be used to control the operation of an electronic device according to an embodiment. The processing circuit units may be based on one or more microprocessor(s), microcontroller(s), digital signal processor(s), baseband processor(s), power management section(s), audio chip(s), application-specific integrated circuit(s), or the like.

According to an embodiment, the memories 12 and 22 may include memory areas for one or more processors 11 and 21 to store variables used in the protocol, configuration, control, and other functions of the device, including operations corresponding to or including any of the methods and/or procedures described as examples in the disclosure. In addition, the memories 12 and 22 may include non-volatile memory, volatile memory, or a combination thereof. Also, the memories 12 and 22 may interface with a memory slot allowing insertion and removal of removable memory cards of one or more formats (e.g., SD card, Memory Stick, Compact Flash, or the like).

According to an embodiment, the transceivers 13 and 23 may include a wireless communication module or a radio-frequency (RF) module. The wireless communication module may include, for example, Wi-Fi, Bluetooth (BT), Global Positioning System (GPS), or Near-Field communication (NFC). For example, the wireless communication module may provide a wireless communication function by using radio frequencies. Additionally or alternatively, the wireless communication module may include a network interface of modem for connecting a robot to a network (e.g., the Internet, a local area network (LAN), a wide area network (WAN), a telecommunication network, a cellular network, a satellite network, a plain old telephone service (POTS), the fifth-generation (5G) network, or the like). The RF module may be responsible for transmitting and receiving data, for example, RF signals or called electronic signals. For example, the RF module may include a power amplifier module (PAM), a frequency filter, a low-noise amplifier (LNA), or the like. In addition, the RF module may further include components for transmitting and receiving electromagnetic waves in free space in wireless communication, for example, a conductor, a wire, or the like.

The blood glucose measurement device 10 according to an embodiment may establish communication based on encrypted images. The encrypted images include, for example, QR code images. The blood glucose measurement device 10 may display a QR code image through a display module, and the user may be linked to a web page for communication setup by capturing an image of the QR code image through a user terminal. The user may fill out a setup application form by using the user terminal and provide the completed application form to a communication station. In various embodiments, NFC and/or BT communication may be used instead of a setup procedure for encrypted images. The user terminal may be linked to a web page for communication setup based on the NFC connection and/or BT connection between the user terminal and the blood glucose measurement device 10. Thereafter, the user may fill out a setup application form and provide the completed application form to a communication station.

Hereinafter, the blood glucose measurement device 10 according to an embodiment is described. The blood glucose measurement device 10 applied to various embodiments may be divided into an invasive device and a non-invasive device based on whether the device invades the skin of the user. In addition, the blood glucose measurement device 10 may be divided into a sampling blood glucose measurement system and a continuous blood glucose measurement system based on whether the device is attached to the body of the user and is able to continuously measure blood glucose levels. The blood glucose measurement device 10 according to an embodiment is applicable to all of the above devices and is not limited thereto.

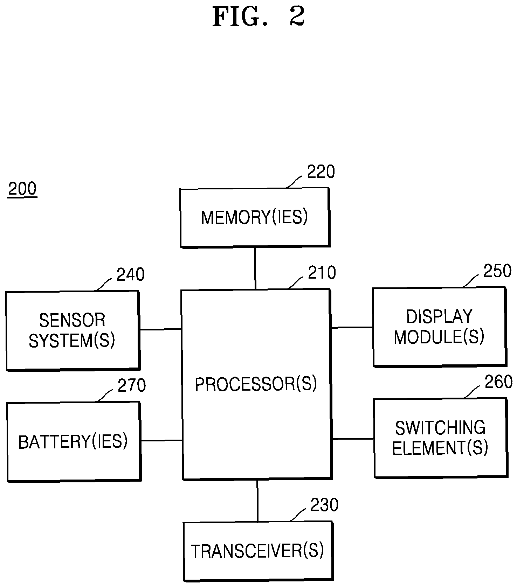

FIG. 2 is a block diagram of a blood glucose measurement device according to an embodiment.

Referring to FIG. 2, a blood glucose measurement device 200 may include one or more processors 210, one or more memories 220, and one or more transceivers 230, and additional descriptions thereof are omitted.

According to an embodiment, the blood glucose measurement device 200 may include a sensor system 240, and the sensor system 240 may include one or more sensors and sensor circuits. A sensor may be configured to electrically couplable to a sensor circuit, and the sensor may be manufactured in a state of being electrically coupled to the sensor circuit, or may be manufactured in a method of being provided at a position that may come into contact with the sensor circuit by an external force. In an operational state in which the blood glucose measurement device 200 is used by a user, the sensor is maintained in electrical contact with the sensor circuit, and a sensing signal (or sensing information) obtained from the sensor is transmitted to the sensor circuit. The sensor according to various embodiments may be provided as an analyte sensor for measuring the user's blood glucose values as a component of the blood glucose measurement device 200, but the disclosure is not limited thereto. In addition, according to an embodiment, the sensor circuit may be implemented as at least a portion of a processing circuit or may be configured in a form electrically coupled to the processing circuit, but is not limited thereto.

According to an embodiment, the blood glucose measurement device 200 may include a display module 250. The processor 210 may output information related to a measured blood glucose value through the display module 250. The information related to a blood glucose value may include, for example, diagnostic information determined based on the measured blood glucose value. The diagnostic information may be generated by one or more processors 210 provided in the blood glucose measurement device 200 or may be generated by one or more processors 210 provided in an external terminal (refer to 20 of FIG. 1) capable of communication with the blood glucose measurement device 200. The external terminal 20 (refer to FIG. 1) refers to various communication-capable devices including, for example, mobile terminals, personal computers (PC), cloud servers, edge computing servers, or the like. The external terminal 20 (refer to FIG. 1) may also correspond to any one of the user terminals described above in FIG. 1.

According to an embodiment, when information to be displayed on the display module 250 is generated by the external terminal 20 (refer to FIG. 1), the blood glucose measurement device 200 may transmit sensing information obtained by the sensor system 240 to the external terminal 20 (refer to FIG. 1), and the external terminal 20 (refer to FIG. 1) may generate information related to a blood glucose value based on the sensing information. The external terminal 20 (refer to FIG. 1) may transmit the generated information related to the blood glucose value to the blood glucose measurement device 200, and the blood glucose measurement device 200 may display the obtained information through the display module 250.

According to an embodiment, the blood glucose measurement device 200 may generate information to be displayed on the display module 250 regardless of the external terminal 20 (refer to FIG. 1). The blood glucose measurement device 200 may generate information related to a blood glucose value based on sensing information obtained by the sensor system 240. The blood glucose measurement device 200 may display information related to a blood glucose value through the display module 250 provided therein.

According to an embodiment, the display module 250 may include a touch screen panel for interacting with the user. The touch screen panel refers to a display panel including a resistive or capacitive touch sensor. The user may apply an input to the touch screen panel, and the display module 250 transmits the touch input to the processor 210. The processor 210 interacts with the user based on the user's touch input. As such, the blood glucose measurement device 200 according to an embodiment may output information related to a blood glucose value and also interact with the user based on the user's touch input.

The display module 250 applied to an embodiment may include a flat display or a flexible display. The flat display is manufactured by using an inflexible substrate, and conversely, the flexible display is manufactured by using a flexible substrate. The flexible display includes a bendable display module 250, a rollable display, a foldable display, or the like, but is not limited thereto. The display panel may include or consist of an organic light-emitting diode (OLED), a liquid crystal display (LCD), an electrochromic display (ECD), an electrophoretic display (EPD), or the like, and the above examples are distinguished based on the type of display panel.

The display module 250 according to an embodiment may output an application execution screen by the processor 210. The execution screen may include at least a portion of a user interface representation associated with an application stored in the memory 220. A portion of the user interface representation may include one or more virtual control buttons. The control buttons may be represented, for example, as graphical elements on the user interface representation. When a user input to a control button is received, the processor 210 may support to perform a corresponding control operation, and in some cases, the user interface representation displayed on the display module 250 may be switched to a different user interface representation.

The application according to an embodiment may include a blood glucose measurement application and/or a blood glucose management application. The blood glucose measurement application may be included as at least a portion of the blood glucose management application but is not limited thereto.

According to an embodiment, the blood glucose measurement device 200 may further include a battery 270. The battery 270 may be electrically connected to at least some of the components of the blood glucose measurement device 200 and supply power to each component. The blood glucose measurement device 200 including the battery 270 may operate electrically even when not being electrically connected to an external power source. That is, the blood glucose measurement device 200 according to an embodiment may be used as a portable device.

According to an embodiment, the blood glucose measurement device 200 may include one or more switching elements 260. The switching element 260 according to an embodiment may include, for example, an operating switch and/or a transfer switch.

According to an embodiment, the operating switch refers to the switching element 260 for activating at least one component of the blood glucose measurement device 200. The operating switch may be controlled to a turn-on state or a turn-off state. The operating switch may connect two or more nodes in a circuit in the turn-on state and open two or more nodes in the turn-off state. For example, the operating switch may be electrically connected to the battery 270 to control the supply and cut-off of power. As another example, the operating switch may be electrically connected to the sensor system 240 to control the activation and inactivation of the sensor system 240. As another example, the operating switch may be electrically connected to the transceiver 230 to control activation or inactivation of a communication function. The operating switch according to an embodiment may be understood to include a combination of an electronic element on a circuit provided inside the blood glucose measurement device 200, such as a processing circuit and a sensor circuit, or a physical button provided on the outside of the device to enable an interaction with the user, and the switching element 260 connected to the electronic element or the physical button.

The operating switch applied to various elements may be activated by another component inserted into the housing of the blood glucose measurement device 200. The operating switch may be activated in response to the insertion of another component (e.g., a strip, an introducer needle) into the blood glucose measurement device 200. For example, the processor 210 may switch the operating switch from a turn-off state to a turn-on state in response to the insertion of the strip into the blood glucose measurement device 200. As another example, the processor 210 may switch the operating switch from the turn-off state to the turn-on state in response to the insertion of a needle into the blood glucose measurement device 200. According to an embodiment, when the operating switch is switched to the turn-on state by the insertion of another component, the turn-on state may be maintained even when the other component is separated or removed from the blood glucose measurement device 200. However, according to an embodiment, the processor 210 may also switch the operating switch to the turn-off state in response to the occurrence of a certain turn-off event.

According to an embodiment, the transfer switch refers to the switching element 260 for transmitting a message or request to the external terminal 20 (refer to FIG. 1) by using the one or more transceivers 230 of the blood glucose measurement device 200. In an alternative embodiment, the transfer switch may also be integral with other components instead of being provided as a separate switching element 260. For example, the transfer switch may be integral with the display module 250. In this case, the processor 210 may activate the transceiver 230 to transmit a message in response to the turning-on (or activation) of the display module 250. As another example, the processor 210 may activate the transceiver 230 to transmit a message in response to the display of a certain user interface representation by the display module 250. In other words, the transfer switch may be integral as a function of the processor 210 and the display module 250.

In addition, according to an embodiment, the blood glucose measurement device 200 may also include one or more transfer switches provided as independent switching elements 260. The transfer switch may be electrically connected to at least some of one or more components of the blood glucose measurement device 200. For example, the transfer switch may be connected between the transceiver 230 and the battery 270 to electrically connect the transceiver 230 to the battery 270. The processor 210 may electrically couple the battery 270 to the transceiver 230 or open the coupling by operating the transfer switch. The transceiver 230 may remain activated while the electrical coupling between the transceiver 230 and the battery 270 is maintained, and the transceiver 230 may not communicate with an external device in an inactivated state while the electrical coupling is opened.

The blood glucose measurement device 200 according to an embodiment may selectively activate the transceiver 230 through the transfer switch. The consumption of the battery 270 inevitably increases while the transceiver 230 and an RF antenna maintain a communication state. However, the blood glucose measurement device 200 according to an embodiment may reduce the consumption of the battery 270 by selectively operating the transceiver 230. In the case of a portable blood glucose measurement device 200 (in particular, a continuous glucose monitoring system (CGMS)), it is important that the battery 270 lasts a long time, and the device according to an embodiment may significantly improve power management efficiency and blood glucose monitoring efficiency.

The blood glucose measurement device 200 according to an embodiment may further include a turn-on switch (not shown) for turning on the blood glucose measurement device. The turn-on switch may be understood as a power button of an electronic device. One or more components of the blood glucose measurement device 200 may be electrically activated based on a user input to the power button. The power button may be provided separately on one side of the housing of the blood glucose measurement device, or may be formed integrally with the display module 250 and/or other switching elements 260.

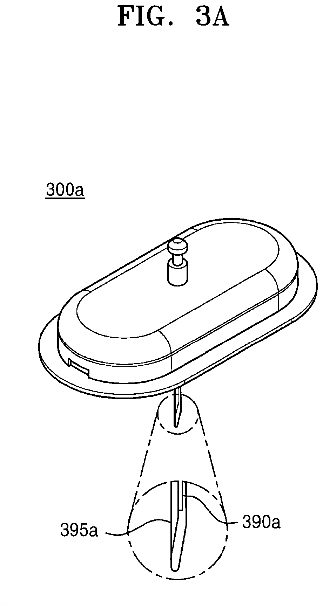

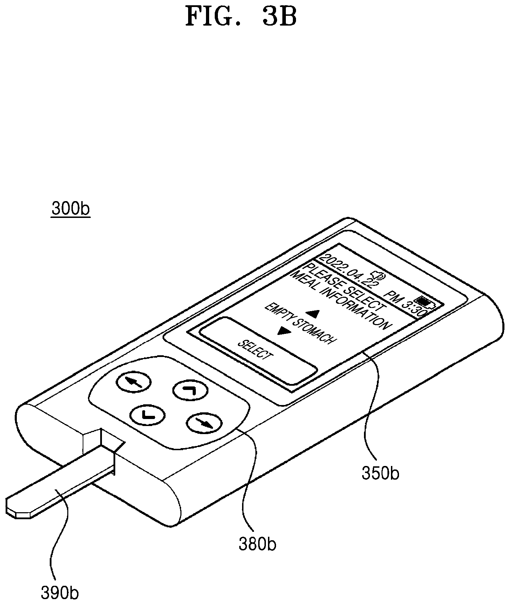

FIGS. 3A and 3B show implementation examples of a blood glucose measurement device. In more detail, FIGS. 3A and 3B illustrate examples of a patch-type device and a strip-type device as implementation examples of a blood glucose measurement device, respectively.

A patch-type device 300a is configured to continuously measure blood glucose, which is attached to the inside of a user's body and may periodically or continuously obtain blood glucose values through an analyte sensor 390a invading the lower surface of the user's skin. A strip-type device 300b includes a space on one side of the housing thereof to accommodate a strip 390b, and blood glucose values may be obtained by applying blood to the strip 390b while the strip 390b is maintained in a state of being attached to the inside of the device.

According to an embodiment, the housing of the patch-type device 300a has one or more openings for receiving an external element (e.g., an introducer needle 395a) penetrating from the outside. The housing may include two openings through which an external element penetrates in a longitudinal direction, and the external element 395a may completely penetrate the patch-type device 300a by penetrating the two holes. Here, the external element 395a may penetrate the patch-type device 300a to introduce a sensor (e.g., the analyte sensor 390a) provided inside the device to the bottom of the skin.

According to an embodiment, the housing of the strip-type device 300b has one or more openings for accommodating an external element (e.g., the strip 390b). The housing may have one opening to accommodate the external element 390b in a longitudinal direction, and the external element 390b may be accommodated inside the housing and electrically coupled to the device. When blood is applied to one side of the strip 390b while the coupling between the device and the external element 390b is maintained, the device 300b may obtain a blood glucose value by analyzing components of the blood. In particular, according to an embodiment, the strip-type device 300b may include one or more control pads 380b. The control pad 380b may be implemented with one or more physical keys to receive a user input. The control pad 380b may be provided separately from a display module 350b, and the control pad 380b and the display module 350b may also be configured to receive user inputs respectively. In some embodiments, the control pad 380b may be understood as one or more virtual key buttons displayed on the display module 350b. For reference, in the disclosure, a 'strip' is for blood glucose measurement and may also be mutually referred to as a 'sensor strip.'

As described above, a blood glucose measurement device according to an embodiment has one or more openings to accommodate an external element or allow the external element to penetrate. In addition, the blood glucose measurement device may have one or more units to detect the accommodation or penetration of an external element. For example, the blood glucose measurement device may include one or more contact electrodes (or induction electrodes). The contact electrodes (not shown) may be electrically coupled to an external element. One or more processors of a blood glucose measurement device may identify an electrical signal based on an electrical coupling between a contact electrode and an external element. In other words, the one or more processors may identify the insertion or penetration of the external element by using one or more contact electrodes.

The blood glucose measurement device according to an embodiment may perform one or more operations in response to the electrical coupling to the external element, and may also perform one or more other operations in response to an interaction with another element regardless of the external element. That is, the blood glucose measurement device according to an embodiment may estimate an intention of the user based on an input unit and provide the user with information appropriate to the estimated intention. A detailed description is given below.

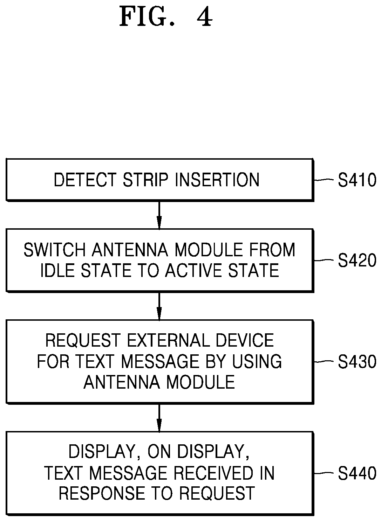

FIG. 4 is a flowchart illustrating a blood glucose measurement method according to an embodiment.

Referring to FIG. 4, a method according to an embodiment may include operation S410 of detecting strip insertion, operation S420 of switching an antenna module from an idle state to an active state, operation S430 of requesting a server for a text message by using the antenna module, and operation S440 of displaying, on a display, a text message received in response to the request. Each operation may be implemented by one or more processors provided in the blood glucose measurement device and at least some other components electrically coupled to the one or more processors.

According to operation S410, one or more processors may identify strip insertion (or penetration) based on an electrical signal generated by electrical coupling between a contact electrode and an external element. One or more electrodes for electrical coupling to the blood glucose measurement device may be provided in advance on one end of the strip. The one or more processors may identify that the strip has been inserted as one end of the strip is electrically coupled to the contact electrode of the housing of the blood glucose measurement device.

The one or more processors may obtain sensing information about blood applied on the strip while the strip is maintained in being inserted into the blood glucose measurement device. According to an embodiment, the sensing information is blood glucose measurement data (blood glucose data) obtained by one or more sensor systems provided in the blood glucose measurement device. For example, in the case of a strip-type device, a sensor system may include a strip and a sensor circuit electrically connected to the strip, and one or more processors may obtain sensing information about blood applied on the strip by using the sensor system. The sensing information obtained in this way may be output through a display module provided in the blood glucose measurement device or transmitted to a communicatively-connected external terminal.

According to operation S420, the one or more processors may switch the antenna module from an idle state to an active state in response to the identification of strip insertion. Here, the antenna module may be interchanged with a transceiver and may include, for example, an RF antenna, an NFC antenna, and a BT antenna, but is not limited thereto. The antenna module may be maintained in an open state with a battery until strip insertion is identified, and may be electrically coupled to the battery when strip insertion is identified. In other words, according to an embodiment, the one or more processors may operate one or more switching elements in response to identification of strip insertion, and the battery and the antenna module may be electrically coupled to each other as the one or more switching elements are operated. As such, the blood glucose measurement device according to an embodiment may electrically activate or inactivate the antenna module based on a coupling relationship between a strip and a contact electrode, thereby preventing battery power from being consumed by the antenna module in unnecessary situations.

According to operation S430, the one or more processors may request the external terminal for a text message by using the antenna module while the antenna module is supplied with power. Here, the term 'request' may be interchangeably used as a 'request message' or 'a message including a request.'

According to an embodiment, the request transmitted to the external terminal may include one or more pieces of sensing information. Here, the sensing information refers to data related to a blood glucose value obtained by the sensor system. In addition, the request transmitted to the server may further include user information. The user information may further include display information of the blood glucose measurement device and the user's status information.

The status information may include various pieces of information related to the user. The status information may include at least one of eating statuses, meal times, and underlying diseases. The eating statuses may include, for example, a status before a meal and a status after a meal. The meal times may include, for example, breakfast, lunch, dinner, and before bedtime. The underlying diseases may include, for example, high blood pressure, low blood pressure, and diabetes. In this way, the various pieces of status information may be used when generating diagnostic information together with sensing information. The status information may be transmitted to the external terminal separately or together with the sensing information to generate diagnostic information.

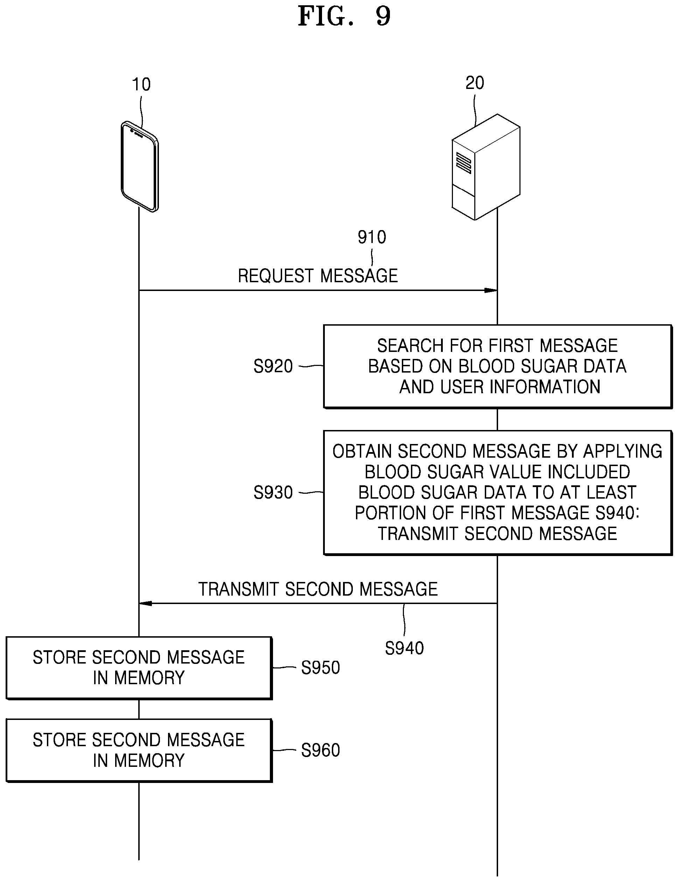

Display information includes one or more preset parameters with respect to a method of outputting information through the display module. For example, the display information includes at least one of a text size, a font type, and a display dimension of the display on which a feedback message will be displayed. When the display information is included, the external terminal may modify at least a portion of the content of the feedback message that responds to the blood glucose measurement device based on the display information. For example, the external terminal may abbreviate or omit at least a portion of the content of the feedback message so that the feedback message may be entirely displayed on a single screen to fit the display dimension of the blood glucose measurement device. A detailed description is given below with reference to FIG. 9.

According to an embodiment, a request for a feedback message may be transmitted to the external terminal in response to extraction or separation of the strip. The device may be set so that the request for the feedback message is not transmitted to the external terminal while the coupling between the strip and the device is maintained. The one or more processors may transmit the request for the feedback message to the external terminal by using the antenna module when electrical separation of the strip and the contact electrode is identified. The antenna module may be supplied with power in response to the identification of strip insertion, but power supply to the antenna module may be maintained for a certain period of time even when the strip is extracted or separated. However, when the certain period of time has elapsed, the one or more processors may switch the antenna module to an idle state or cut off power supply to the antenna module.

According to operation S440, the one or more processors may output the feedback message received from the external terminal on the display. The feedback message may include diagnostic information for the user. The user may easily determine an action related to health maintenance, such as visiting the hospital, by checking the feedback message.

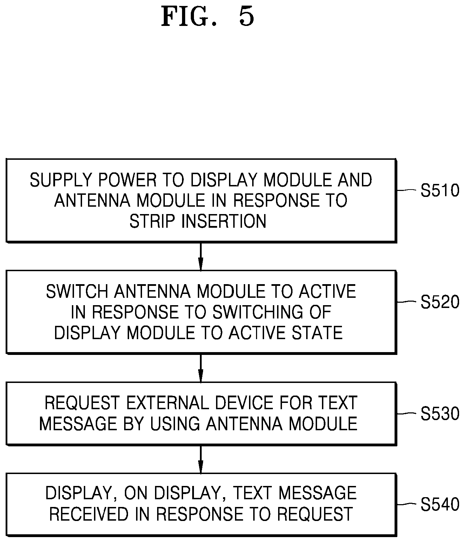

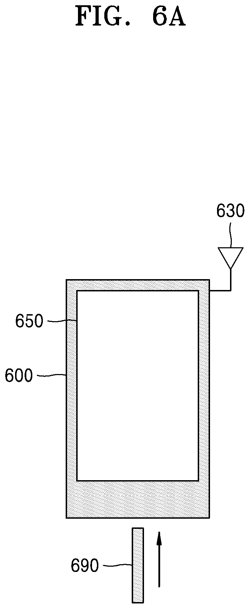

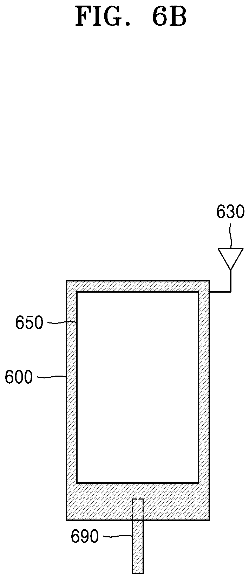

FIG. 5 is a flowchart illustrating a blood glucose measurement method according to another embodiment. FIGS. 6A and 6B are diagrams illustrating an example of a process of activating one or more components of a blood glucose measurement device based on an interaction with an external element.

Referring to FIG. 5, the blood glucose measurement method according to an embodiment includes operation S510 of supplying power to a display module and an antenna module in response to identification of strip insertion, operation S520 of switching the antenna module to an active state in response to switching the display module to an active state, operation S530 of requesting an external terminal (e.g., a server) for a feedback message (e.g., a feedback message) by using the antenna module, and operation S540 of displaying, on the display, a feedback message received from the external terminal.

According to operation S510, the one or more processors may supply power to the display module and the antenna module in response to the identification of strip insertion. A battery, the display module, and the antenna module may be maintained in an open state before the strip is inserted. The one or more processors may electrically couple the battery to the display module to supply power to the display module, in response to the identification of strip insertion. In addition, the one or more processors may electrically couple the battery to the antenna module to supply power to the antenna module, in response to the identification of strip insertion. In addition, power supply to the display module and the antenna module may also be performed simultaneously in response to the identification of strip insertion.

To distinguish between power supply and cut-off, one or more switching elements may be provided between the display module and the battery and/or between the antenna module and the battery. The one or more processors may determine whether to supply or cut off power by controlling the one or more switching elements.

According to an embodiment, the supply of power and the switching to an active state may be operationally separated. The one or more processors may output information through the display module while the display module is maintained in the active state. The one or more processors may transmit information through the antenna module while the antenna module is maintained in the active state.

The one or more processors may switch the display module and/or the antenna module between an idle state and an active state through one or more control commands while power supply is maintained. The one or more processors may switch the antenna module from the idle state to the active state in response to switching the display module from the idle state to the active state while power supply to the display module and/or the antenna module is maintained. In other words, the active state of the display module and the active state of the antenna module are operationally linked.

Referring to FIGS. 5, 6A, and 6B, in response to insertion of a strip 690 into a blood glucose measurement device 600, one or more processors of the blood glucose measurement device 600 may supply power to a display module 650 or switch the display module 650 from an idle state to an active state while power supply is maintained. Referring to FIG. 6A, before the strip 690 is inserted into the blood glucose measurement device 600, power is not supplied to the display module 650, or the display module 650 is maintained in the idle state even when power supply is maintained. As shown in FIG. 6B, when the strip 690 is inserted into the blood glucose measurement device 600, the one or more processors of the blood glucose measurement device 600 may supply power to the display module 650 or switch the display module 650 from the idle state to the active state while power supply is maintained. An operation of supply power to the display module 650 and an operation of switching from an idle state to an active state duplicate the descriptions with respect to the above embodiments, and thus the descriptions thereof are omitted.

Referring to FIG. 6A, before the strip 690 is inserted into the blood glucose measurement device 600, power is not supplied to an antenna module 630, or the antenna module 630 is maintained in an idle state even when power supply is maintained. As shown in FIG. 6B, when the strip 690 is inserted into the blood glucose measurement device 600, the one or more processors of the blood glucose measurement device 600 may supply power to the antenna module 630 or switch the antenna module 630 from an idle state to an active state while power supply is maintained.

In addition, the one or more processors may supply power to the antenna module 630 at the same time as supplying power to the display module 650 or immediately after supplying power to the display module 650. Alternatively, when the display module 650 is switched from the idle state to the active state while power supply to the display module 650 is maintained, the one or more processors may supply power to the antenna module 630. The power supply may be performed, for example, by operating one or more switching elements. The one or more processors may switch the antenna module 630 from the idle state to the active state or from the active state to the idle state while power is supplied to the antenna module 630.

According to operation S530, the one or more processors may request the external terminal (e.g., a server) for a text message by using the antenna module while the antenna module is maintained in the active state. The request may include at least one of sensing data, status information, and display information as described above in operation S430.

According to operation S540, the one or more processors may display, on the display, a text message received in response to the request.

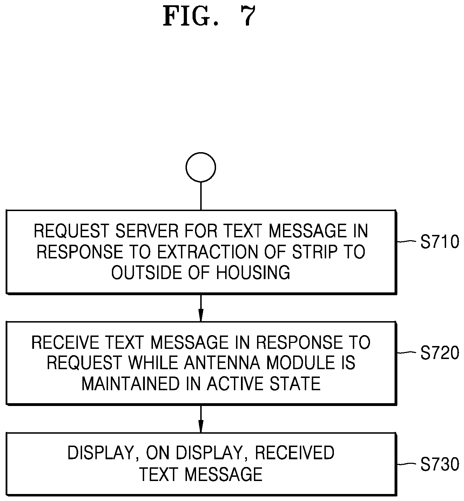

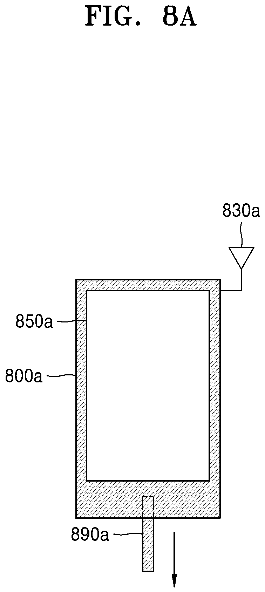

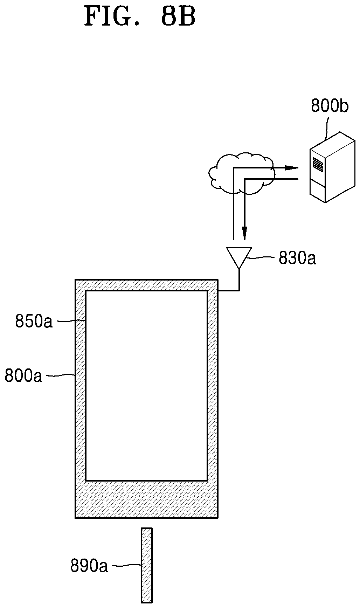

FIG. 7 is a flowchart for describing operation S430 of FIG. 4 and operation S530 of FIG. 5. FIGS. 8A and 8B are diagrams illustrating an example of a process of transmitting a feedback message request to an external device based on an interaction with an external element.

Referring to FIG. 7, operation S430 of FIG. 4 and operation S530 of FIG. 5 include operation S710 of requesting an external terminal for a text message in response to extraction of strip outside a housing, operation S720 of receiving a text message in response to the request while an antenna module is maintained in an active state, and operation S730 of displaying the received text message on a display.

According to operation S710, one or more processors of a blood glucose measurement device may control the antenna module to transmit a certain request in response to the extraction of strip outside the housing. The one or more processors may identify that the strip is extracted from the inside of the housing based on a change in electrical signals caused by an opening of an electrical coupling between one or more contact electrodes and the strip. When separation of the strip from the housing is identified, the one or more processors may control the antenna module to transmit a transmission request for a text message to the external terminal. The antenna module may be switched to an active state in response to insertion of a strip and may be maintained in the active state for a certain period of time even when the strip is separated from the housing. Alternatively, the antenna module may also be maintained in the active state until the display module is switched to an idle state or power supply to the display module is blocked even when the strip is separated from the housing. For reference, the term 'extraction' in the disclosure may be interchangeably used with 'separation.'

Referring to FIGS. 7, 8A, and 8B, it may be understood that a blood glucose measurement device 800a requests an external terminal 800b for a text message based on extraction of a strip 890a. Referring to FIG. 8A, a user may remove the strip 890a from the blood glucose measurement device 800a. A display module 850a may be maintained in an active state until the strip 890a is removed. Referring to FIG. 8B, one or more processors of the blood glucose measurement device 800a may transmit a transmission request for a text message to the external terminal 800b (e.g., a remote control server 800b) through an antenna module 830a, in response to removal of the strip 890a. The one or more processors may change a user interface representation displayed through the display module 850a in response to the removal of the strip 890a. For example, the one or more processors may control the display module 850a to display a user interface representation showing a blood glucose measurement result until the strip 890a is removed and may control the display module 850a to display a user interface representation informing message transmission when the strip 890a is removed.

According to various embodiments, a transmission request for a text message may also be transmitted to the external terminal 800b even when the strip 890a is not separated or removed. When the strip 890a is not removed until a certain period of time has elapsed from a time point at which sensing information is obtained by the strip 890a, the one or more processors of the blood glucose measurement device 800a may transmit a transmission request for a text message to the external terminal 800b regardless of the separation or removal of the strip.

FIG. 9 is a sequence diagram of a blood glucose measurement method according to an embodiment.

Referring to FIG. 9, a blood glucose measurement method performed by a blood glucose measurement system includes operation S910 in which the blood glucose measurement device requests an external terminal for a message, operation S920 in which the external terminal searches for a first message based on blood glucose data and user information, operation S930 in which the external terminal obtains a second message by applying a blood glucose value to at least a portion of the first message, operation S940 in which the external terminal transmits the second message to the blood glucose measurement device, operation S950 in which the blood glucose measurement device stores the received second message in a memory, and operation S960 in which the blood glucose measurement device displays the second message on a display. Descriptions already given with reference to FIGS. 4 to 8B are omitted, and additional embodiments are mainly described. Operation S910 is identical or equivalent to operation S430 of FIG. 4 and operation S530 of FIG. 5, and operations S950 and S960 are identical or equivalent to operation S440 of FIG. 4 and operation S540 of FIG. 5.

According to operation S910, the blood glucose measurement device may request the external terminal for a text message by using an antenna module while power is supplied to the antenna module.

According to an embodiment, the request transmitted to the external terminal may include one or more pieces of sensing information. Here, the sensing information refers to data related to a blood glucose value obtained by the sensor system. In addition, the request transmitted to a server may further include user information. The user information may further include display information of the blood glucose measurement device and the user's status information.

The status information may include various pieces of information related to the user. The status information may include at least one of eating statuses, meal times, and underlying diseases. The eating statuses may include, for example, a status before a meal and a status after a meal. The meal times may include, for example, breakfast, lunch, dinner, and before bedtime. The underlying diseases may include, for example, high blood pressure, low blood pressure, and diabetes. In this way, the various pieces of status information may be used when generating diagnostic information together with sensing information. The status information may be transmitted to the external terminal separately or together with the sensing information to generate diagnostic information.

The display information includes one or more preset parameters with respect to a method of outputting information through the display module. For example, the display information includes at least one of a text size, a font type, and a display dimension of the display on which a feedback message will be displayed. When the display information is included, the external terminal may modify at least a portion of the content of the feedback message that responds to the blood glucose measurement device based on the display information. For example, the external terminal may abbreviate or omit at least a portion of the content of the feedback message so that the feedback message may be entirely displayed on a single screen to fit the display dimension of the blood glucose measurement device.

According to operation S920, the external terminal may search for the first message based on sensing information (e.g., the blood glucose data) and the user information. According to an embodiment, a plurality of text messages are recorded in a memory of the external terminal. The plurality of text messages may include or consist of a first text message group and a second text message group. The first text message group may include a message informing any one of normal blood glucose, high blood glucose, and low blood glucose. The second text message group includes a message informing that a blood glucose value is less than a minimum value of a target range by comparing the blood glucose value with the target range, a message informing that the blood glucose value is within the target range, and a message informing that the blood glucose value exceeds a maximum value of the target range.

According to an embodiment, the external terminal may select any one of messages in the first text message group and any one of messages in the second text message group based on the sensing information and a preset target range. The external terminal may generate a feedback message by combining the message selected from the first text message group and the message selected from the second text message group.

According to an embodiment, a first text message and a second text message may have different contents. According to an embodiment, when context information of the first text message does not match with context information of the second text message, the external terminal may correct the feedback message so that the context information of the first text message matches with the context information of the second text message. In a correction operation, the context information with respect to the second text message is set to have higher priority than the context information with respect to the first text message.

In addition, according to an embodiment, the request mentioned in operation S910 (or operation S430 of FIG. 4 and operation S530 of FIG. 5) may further include preset display information of the blood glucose measurement device, and the context of the feedback message may also be corrected based on the preset display information. The external terminal may modify the context of the feedback message based on at least one of a text size, a font type, and a display dimension of the display on which the feedback message will be displayed. In other words, the character count and expression of the content of the feedback message may be changed so that the feedback message may be entirely displayed through a single screen. The external terminal may correct the context of the feedback message so that the feedback message is displayed with a single screen while maintaining a preset text size when the feedback message is displayed through the display module of the blood glucose measurement device.

The feedback message generated in this way may be transmitted from the external terminal to the blood glucose measurement device, stored in the memory of the blood glucose measurement device, and displayed through the display module of the blood glucose measurement device (operations S950 and S960).

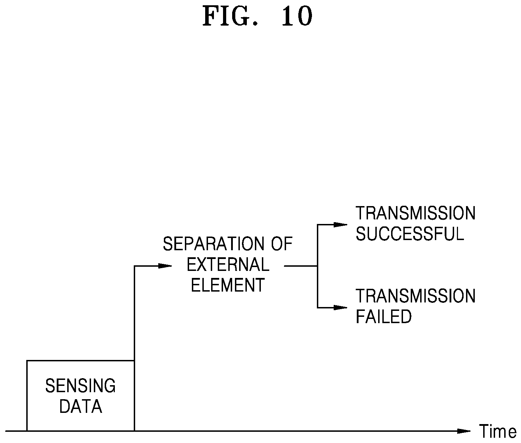

A process of generating a feedback message based on a network communication between a blood glucose measurement device and an external terminal is described above. However, the antenna module of the blood glucose measurement device is not always maintained in an active state, and in some cases, the network connection may be unstable, and thus a feedback message may not be received or a request for the feedback message may not be transmitted. Hereinafter, a method of requesting a feedback message adaptively to a network environment is described with reference to FIGS. 10 to 13.

Referring to FIG. 10, a request for a text message transmitted to an external terminal may include sensing information (or sensing data). According to an embodiment, the sensing data may be transmitted to the external terminal based on separation or removal of an external element. Here, the transmission may succeed or fail based on a network condition.

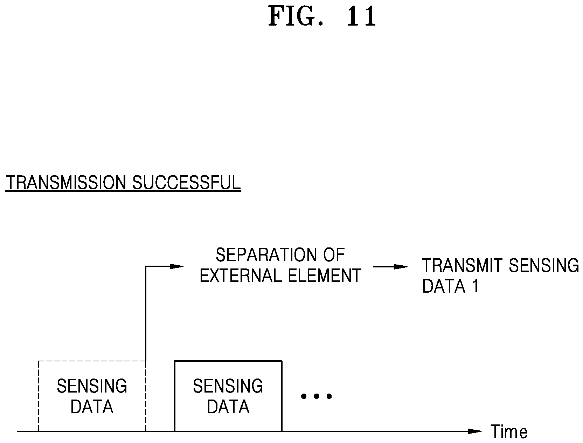

Referring to FIG. 11, the request may include one or more pieces of sensing information. When the transmission of the request to the external terminal is successful, at least a portion of the transmitted sensing information may be excluded from a memory.

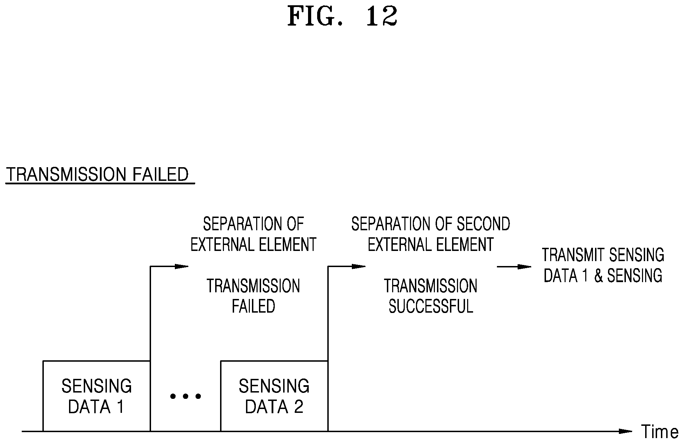

Referring to FIG. 12, the request transmitted to the external terminal may also include two or more pieces of sensing information. The sensing information may be accumulated whenever a blood glucose value is obtained by a sensor system (e.g., a sensor, a sensor strip, and an analyte sensor) of the blood glucose measurement device.

In the case of a strip-type device, the sensing information may be obtained once per strip. The sensing information stored in the memory is transmitted to the external terminal whenever a strip is removed from the blood glucose measurement device, and the transmitted sensing information is excluded from the memory, and thus the sensing information is not typically accumulated. However, when the sensing information may not be transmitted to the external terminal, the sensing information that failed transmission may remain recorded in the memory, and as a result, a plurality of pieces of sensing information may be stored in the memory.

In the case of a patch-type device, the sensing information may be continuously or periodically obtained while the blood glucose measurement device is attached to the body. Accordingly, a plurality of pieces of sensing information may be stored in a memory.

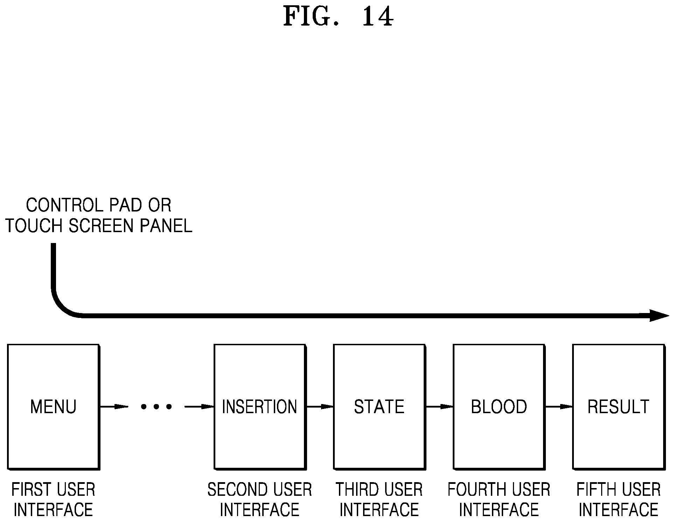

According to an embodiment, the sensing information may be transmitted to the external terminal in response to removal of an external element (e.g., a strip). According to another embodiment, the sensing information may also be transmitted to the external terminal based on an operation of the display module. For example, the sensing information may be transmitted to the external terminal based on the switching of the display module from an idle state to an active state. As another example, the sensing information may also be transmitted based on switching of a user interface representation being displayed on the display module. An operation of transmitting sensing information based on switching of a user interface representation is described below with reference to FIGS. 14 to 16.

Referring to FIG. 12 again, transmission of first sensing information, which is firstly obtained, to the external terminal may be attempted in response to removal of an external element from the blood glucose measurement device by a user. When the transmission of the first sensing information to the external terminal is failed, the first sensing information remains recorded in the memory. Thereafter, the user may obtain second sensing information temporally later by using another external element, and the second sensing information may be transmitted to the external terminal in response to removal of the other external element from the device. According to an embodiment, the blood glucose measurement device may include the second sensing information and the first sensing information stored in the memory in a request and transmit the request to the external terminal in response to the removal of the other external element.

In the above embodiment, the second sensing information may be understood as sensing information obtained at a time point adjacent to a time point at which transmission of a request was triggered most recently. In the above embodiment, the first sensing information may be understood as one or more pieces of sensing information obtained temporally prior to the second sensing information, and the first sensing information may include or consist of one or two or more pieces of sensing information.

In some embodiments, when a plurality of pieces of sensing information is stored in the memory, the sensing information transmitted to the external terminal may be determined by selecting at least some of the plurality of pieces of sensing information. For example, the blood glucose measurement device may select and transmit at least some of the plurality of pieces of sensing information, the at least some of the plurality of pieces of sensing information, which is transmitted, may be excluded from the memory, and sensing information, which is not transmitted, may be maintained in a recorded state in the memory. According to an embodiment, the sensing information to be transmitted to the external terminal may be determined based on at least one of an obtaining time of blood glucose information or a transmission status whether to transmit the sensing information. The obtaining time refers to a time at which sensing information is obtained. The transmission status is information indicating whether to transmit sensing information. Sensing information already transmitted to the external terminal may be classified as 'transmitted,' and sensing information that has no history of being transmitted to the external terminal may be classified as 'non-transmitted.'

According to an additional embodiment, at least a portion of sensing information transmitted to the external terminal will be deleted from the memory, but the remaining portion thereof remains in the memory based on a certain condition. As such, the sensing information that remains recorded in the memory may be coupled to a tag indicating already transmitted information to be distinguished from non-transmitted sensing information. Here, the certain condition may include a time condition and a number condition of the sensing information. The time condition is related to a time at which sensing information is obtained, and sensing information within a certain time range from a date on which the sensing information is obtained may be maintained in the memory even after being transmitted to the external terminal. The number condition is the minimum number of pieces of sensing information to be remained in the memory. For example, when the number condition is set to 2, at least two pieces of sensing information should be recorded in the memory. In this case, at least some pieces of sensing information that have already been transmitted are maintained in the memory in a state of being coupled to a tag so that the minimum number of pieces of sensing information may be maintained in the memory even after the sensing information is transmitted to the external terminal.

Referring to FIGS. 9 to 12 again, when a plurality of pieces of sensing information are received, the external terminal may generate a feedback message by using at least some of the plurality of pieces of sensing information. In an embodiment, the external terminal may select at least some pieces of sensing information based on an obtaining time of each piece of sensing information and generate a feedback message based on the at least some selected pieces of sensing information. For example, the external terminal may generate a feedback message based on sensing information that is the most recently obtained. As another example, the external terminal may generate a feedback message by selecting sensing information that is the most recently obtained and at least one piece of sensing information that is temporally adjacent to the most recently obtained sensing information. Here, the number of pieces of at least one adjacent piece of sensing information may be determined based on preset setting. When a feedback message is generated by using two or more pieces of sensing information, diagnostic information based on a trend generated by the two or more pieces of sensing information may be further included in the feedback message. The external terminal may generate a trend by using two or more pieces of sensing information and generate a feedback message based on the two or more pieces of sensing information and/or user information (or user data).

In some embodiments, transmission of sensing information stored in the memory may also be performed based on a particular user input. When the particular user input is received, one or more processors of the device may transmit sensing information to the external terminal by using the antenna module regardless of whether a user interface representation is switched, whether an external element is inserted/removed, and a state of the display module. The particular user input may be received through a control pad and/or touch screen pad provided on the blood glucose measurement device.

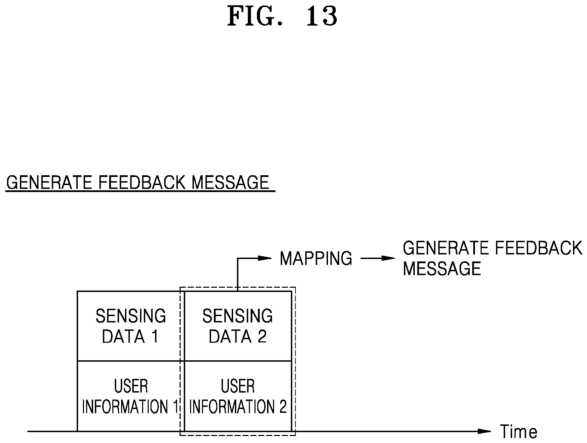

Referring to FIG. 13, in an operation of generating a feedback message, a request transmitted to the external terminal may further include user information in addition to sensing information. The sensing information and the user information may be mapped to be related to each other. For example, first sensing information may be mapped to be related to first user information, and second sensing information may be mapped to be related to second user information. The first user information may be generated based on a user input to the blood glucose measurement device before the first sensing information is obtained. The second user information may be generated based on a user input to the blood glucose measurement device before the second sensing information is obtained. Accordingly, a set configured by the first sensing information and the first user information may be distinguished from a set configured by the second sensing information and the second user information based on an obtaining time.

The blood glucose measurement device may transmit at least some pieces of the first and second sensing information and/or the first and second user information to the external terminal to generate a feedback message. For example, the blood glucose measurement device may select and transmit the first sensing information and the first user information related to the first sensing information to the external terminal, or may select and transmit the second sensing information and the second user information related to the second sensing information to the external terminal. At the same time or immediately thereafter, the blood glucose measurement device may request the external terminal for generation of a feedback message.

The blood glucose measurement device according to an embodiment may supply power to another component, cut off power, or switch the other component to an active state based on insertion and separation of an external element, such as a strip.

In particular, the blood glucose measurement device according to an embodiment may include a touch screen panel for receiving a user's electrostatic touch input or a control pad for receiving a physical key input of the user. The blood glucose measurement device according to an embodiment distinguishes a first interaction input through the touch screen panel and/or the control pad from a second interaction electrically coupled to an external element to display different user interface representations. As such, the blood glucose measurement device according to an embodiment may provide a user-friendly user interface environment by displaying different user interface representations based on an interaction routes, and a process of switching a user interface representation is dividedly described according to types of interactions with reference to FIGS. 14 to 16.

FIG. 14 is a diagram illustrating a process of switching a user interface representation based on a first interaction through a control pad and/or a touch screen panel.

Referring to FIG. 14, a blood glucose measurement device may output a first user interface representation based on a user input through the control pad 380b (refer to FIG. 3) and/or a touch screen panel (a component of 350b of FIG. 3). The first user interface representation includes a menu for calling another user interface representation. The blood glucose measurement device may display, on a display, a second user interface representation informing insertion of an external element (e.g., a strip) based on one or more user inputs to the first user interface representation. When insertion of the external element into a housing is identified, the blood glucose measurement device may display a third user interface representation for inputting user information. While the third user interface representation is being output, the blood glucose measurement device may obtain user information by receiving a user input through the control pad or the touch screen panel. In some embodiments, the third user interface representation is a touch-sensitive interface representation, which may receive one or more touch inputs for inputting user information through the touch screen panel. The blood glucose measurement device may display a fourth user interface representation that requests application of a blood sample to the external element in response to termination of input of user information through the third user interface representation. When the blood sample is applied to the external element and sensing information with respect to the blood sample is obtained, the blood glucose measurement device may include the sensing information in a fifth user interface representation and display the fifth user interface representation on the display.

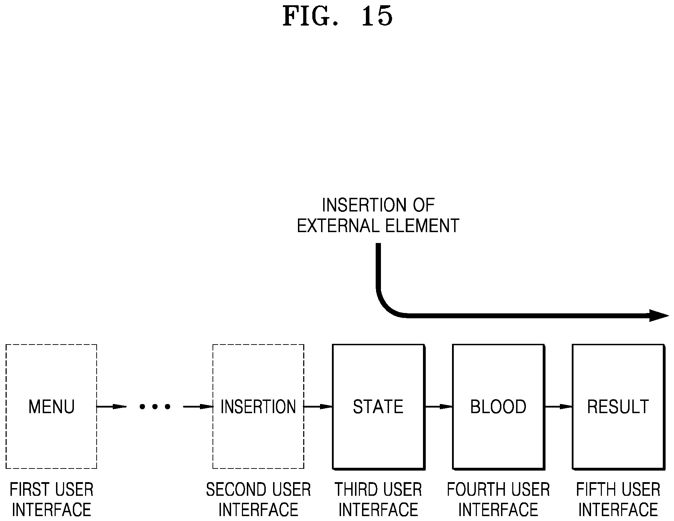

FIG. 15 is a diagram illustrating a process of switching a user interface representation based on a second interaction through an external element.

Referring to FIG. 15, when the second interaction with respect to the external element is identified, a blood glucose measurement device may omit first and second user interface representations and display a third user interface representation on a display. As such, the blood glucose measurement device according to an embodiment may omit at least some of the entire user interface representations based on the first and second interactions and directly call and display an associated user interface representation. This may significantly improve user convenience.

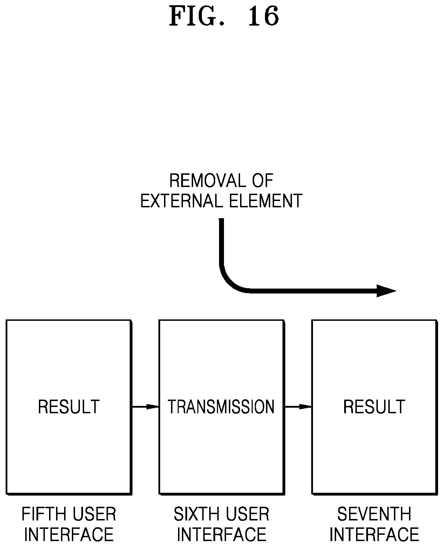

FIG. 16 is a diagram illustrating user interface representations in operations of requesting a feedback message and displaying.

Referring to FIG. 16, a user may remove an external element from a blood glucose measurement device while output of a fifth user interface representation is maintained. When separation and removal of the external element from the inside of a housing is identified, the blood glucose measurement device may display a sixth user interface representation on a display.

According to an embodiment, the blood glucose measurement device may switch an antenna module from an idle state to an active state before, simultaneously with, or immediately after the sixth user interface representation is displayed on the display. According to an embodiment, the blood glucose measurement device may switch the antenna module from the idle state to the active state in response to display of the fifth user interface representation on the display. In some embodiments, the antenna module may be switched to the active state in response to insertion of the external element or may be switched to the active state in response to switching of a display module to an active state. However, to further efficiently manage power consumption for the antenna module, the switching of the antenna module to the active state may also be performed based on switching of user interface representation. The antenna module may be maintained in the idle state until sensing information (e.g., a blood glucose value) is displayed through the fifth user interface representation, and the antenna module may be switched to the active state in response to display of the fifth user interface representation.

When the external element is separated and removed from the blood glucose measurement device while the antenna module is maintained in the active state, the fifth user interface representation is changed to a sixth user interface representation, and the sixth user interface representation is displayed. The sixth user interface representation may include or consist of an animation or icon indicating transmission of a message. The blood glucose measurement device may change the sixth user interface representation to a seventh user interface representation and display the seventh user interface representation in response to completion of transmission of a message, that is, a request for a feedback message. The seventh user interface representation includes at least a portion of a feedback message received from the external terminal. The feedback message may be generated based on sensing information, user information, and/or display information, which is transmitted to the external terminal. The feedback message may include or consist of diagnostic information generated based on one or more pieces of information included in the request.

An electronic device according to various embodiments in the disclosure may be various forms of devices. The electronic device may include, for example, a display device, a portable communication device (e.g., a smartphone), a computer device, a portable multimedia device, a portable medical device, a camera, a wearable device, or a home-appliance device. The electronic device according to an embodiment is not limited to the above devices.

Various embodiments and the terms used in the embodiments are not intended to limit the technical features of the disclosure to specific embodiments, and various changes, equivalents, and substitutes of the embodiments are included. For example, an expression used in the singular encompasses the expression of the plural, unless it has a clearly different meaning in the context. The term It should be understood that the term 'and/or' as used in the disclosure includes any and all possible combinations of one or more of the listed items. In the disclosure, it is to be understood that the terms such as "including," "having," and "comprising" are intended to indicate the existence of the features, components, parts, or combinations thereof disclosed in the disclosure, and are not intended to preclude the possibility that one or more other features, components, parts, or combinations thereof may exist or may be added. In the disclosure, each of the phrases such as "A or B," "at least one of A and B," "A, B, or C," "at least one of A, B, and C," and "at least one of A, B, or C" may include any one of the items listed together in the phrase, or all possible combinations of the listed items. The terms "first," "second," etc. may be used herein to distinguish one component from another, and these components should not be limited by these terms in any other aspect (e.g., importance or order).

The term "~unit" or "~module" used in various embodiments of the disclosure may include units implemented in hardware, software, or firmware, and may be interchangeably used with terms, such as logic, logic block, part, or circuit. The term "~unit" or "~module" may be a part that is configured integrally, or a minimum unit or a portion of the part that performs one or more functions. For example, according to an embodiment, the term "~unit" or "~module" may be implemented in an application-specific integrated circuit (ASIC) form.

The term "information" used in various embodiments of the disclosure may be interpreted to mean "data" according to the context. In addition, the term "if" as used in various embodiments in the disclosure may be interpreted to mean "when," "in case of," "in response to determination," or "in response to detection" according to the context. Similarly, the phrase "when it is determined..." or "when it is detected..." may be interpreted to mean "when determining," "in response to determination," "when detecting," or "in response to detection" according to the context.

A program executed by the blood glucose measurement devices 10, 200, 300a, and 300b described in the disclosure may be implemented as a hardware component, a software component, and/or a combination of a hardware component and a software component. The program may be performed by any system that may execute computer-readable instructions.

Software may include a computer program, code, instructions, or a combination of one or more of the above, and may configure a processing device to operate in a desired way, or may independently or collectively command a processing device. Software may be implemented as a computer program including instructions stored in computer-readable storage media. The computer-readable storage media include, for example, magnetic storage media (e.g., ROM, RAM, a floppy disk, a hard disk, or the like), optical readable media (e.g., CD-ROM, a digital versatile disc (DVD)), or the like. The computer-readable storage media may be distributed across network-connected computer systems so that computer-readable codes may be stored and performed in a distributed manner. A computer program may be distributed directly online (e.g., download or upload) through an application store (e.g., Play Store^{™}) or between two user devices (e.g., smartphones). In the case of online distribution, at least a portion of a computer program product may be temporarily stored or temporarily generated in a machine-readable storage medium, such as a server of a manufacturer, a server of an application store, or a memory of an intermediary server.

According to various embodiments, each of the components described above (e.g., modules or programs) may include a plurality of entities, and some of the entities may also be separately arranged in other components. According to various embodiments, one or more components among the components described above or operations or operations may be omitted, or one or more other components or operations may be added. Alternatively or additionally, a plurality of components (e.g., modules or programs) may be integrated into one component. In this case, the integrated component may perform one or more functions of each of the plurality of components identically or similarly to those performed by a corresponding component among the plurality of components before integration. According to various embodiments, operations performed by a module, a program, or another component may be performed sequentially, in parallel, repeatedly, or heuristically, or one or more of the operations may be performed in a different order, omitted, or one or more operations may be added.

## Claims

1. A blood glucose measurement device having a display, comprising:
a housing having one or more openings on one side;
one or more contact electrodes configured to electrically couple with a sensor strip inserted into the housing through the one or more openings;
one or more control buttons configured to turn on the blood glucose measurement device; and
a processor configured to control a first execution screen to be displayed in response to receiving a first signal by the one or more contact electrodes, and display a second execution screen in response to receiving a second signal by the one or more control buttons.

2. The blood glucose measurement device of claim 1, further comprising a control pad configured to receive a control input,
wherein the processor is further configured to display, on the display, a third execution screen in response to receiving one or more control inputs through the control pad while the second execution screen is displayed, wherein the third execution screen is identical to the first execution screen or comprises a user interface representation to output the first execution screen.

3. The blood glucose measurement device of claim 1, wherein the first execution screen comprises a first user interface representation to collect one or more pieces of information for diabetes diagnosis, and the second execution screen comprises a second user interface representation including a menu to switch to a plurality of user interface representations.

4. The blood glucose measurement device of claim 1, further comprising a control pad configured to receive a control input,
wherein the processor is further configured to generate blood glucose information based on at least one of one or more additionally-input control inputs and one or more sensing values detected by using the sensor strip after the first execution screen or the second execution screen is displayed, and
display, on the display, a fourth execution screen indicating the blood glucose information.

5. The blood glucose measurement device of claim 4, further comprising one or more antennas,
wherein the processor is further configured to activate the one or more antennas in response to the display of the fourth execution screen on the display.

6. The blood glucose measurement device of claim 1, further comprising one or more antennas,
wherein the processor is further configured to control the one or more antennas to transmit a message requesting feedback to an external device in response to opening of an electrical coupling between the sensor strip and the one or more contact electrodes.

7. The blood glucose measurement device of claim 6, wherein the processor
is further configured to store blood glucose information obtained by using the sensor strip in a memory,
include, to the message, the blood glucose information stored in the memory and transmit the message to the external device, and
exclude, from the memory, the blood glucose information transmitted to the external device.

8. The blood glucose measurement device of claim 6, wherein the processor
is further configured to store blood glucose information obtained by using the sensor strip in a memory,
select at least a portion of the blood glucose information stored in the memory, based on at least one of an obtaining time of the blood glucose information or a transmission status of the blood glucose information, and include, to the message, the selected portion of the blood glucose information and transmit the message to the external device.

9. A blood glucose measurement device having a display, comprising:
a housing having one or more openings on one side;
one or more induction electrodes configured to electrically couplewith an introducer inserted into the housing through the one or more openings;
one or more control buttons configured to turn on the blood glucose measurement device;
one or more glucose sensors configured to continuously or periodically obtaining blood glucose information; and
one or more processors configured to control a first execution screen to be displayed in response to receiving a first signal generated between the one or more induction electrodes and the introducer, and display a second execution screen in response to receiving a second signal by the one or more control buttons.

10. The blood glucose measurement device of claim 9, wherein the display comprises a touch sensor configured to receive a control input, and
the one or more processors are further configured to display, on the display, a third execution screen in response to receiving one or more control inputs through the touch sensor while the second execution screen is displayed, wherein the third execution screen is identical to the first execution screen or comprises a user interface representation to output the first execution screen.

11. The blood glucose measurement device of claim 9, wherein the first execution screen comprises a first user interface representation to collect one or more pieces of information for diabetes diagnosis, and the second execution screen comprises a second user interface representation including a menu to switch to a plurality of user interface representations.

12. The blood glucose measurement device of claim 9, wherein the display comprises a touch sensor configured to receive a control input, and
the one or more processors are further configured to obtain blood glucose information based on at least one of one or more additionally-input control inputs and one or more sensing values detected by using the one or more glucose sensors, after the first execution screen or the second execution screen is displayed, and
display, on the display, a fourth execution screen indicating the blood glucose information.

13. The blood glucose measurement device of claim 12, further comprising one or more antennas,
wherein the one or more processors are further configured to activate the one or more antennas in response to the display of the fourth execution screen on the display.

14. The blood glucose measurement device of claim 9, further comprising one or more antennas,
wherein the one or more processors are further configured to store, in a memory, one or more pieces of blood glucose information obtained by the one or more glucose sensors, and
include, to a message requesting feedback, at least some of the one or more pieces of blood glucose information stored in the memory and transmit the message to an external device.

15. The blood glucose measurement device of claim 14, wherein the one or more processors are further configured to exclude, from the memory, the blood glucose information transmitted to the external device.

16. The blood glucose measurement device of claim 14, wherein the one or more processors are further configured to select at least some of the one or more pieces of blood glucose information stored in the memory, based on an obtaining time of the blood glucose information or a transmission status of the blood glucose information, and
include, to a message requesting feedback, only the selected blood glucose information and transmit the message to the external device.

17. The blood glucose measurement device of claim 9, wherein the one or more openings are formed to penetrate the housing in a moving direction of the introducer.

18. A blood glucose measurement method performed by a blood glucose measurement device comprising a housing having one or more openings one side, one or more contact electrodes configured to electrically couple with a sensor strip inserted into the housing through the one or more openings, and one or more control buttons configured to turn on the blood glucose measurement device, the blood glucose measurement method comprising:
displaying a first execution screen in response to receiving a first signal by the one or more contact electrodes; and
displaying a second execution screen in response to receiving a second signal by the one or more control buttons.
